# EUROPÄISCHE PATENTSCHRIFT

(11) **EP 2 214 768 B1**
(45) Veröffentlichungstag und Bekanntmachung des Hinweises auf die Patenterteilung: **25.12.2019**
(21) Anmeldenummer: 08772898.6
(22) Anmeldetag: 22.07.2008
(51) Int. Cl.: A61M 25/00, A61F 2/95

(54) **KATHETER**
CATHETER
SONDE

(30) Priorität: 29.10.2007 CH 16752007
(43) Veröffentlichungstag der Anmeldung: 11.08.2010
(73) Patentinhaber: SIS Medical AG, 8500 Frauenfeld (CH)
(72) Erfinder: SCHWAGER, Michael, 8404 Winterthur (CH)
(74) Vertreter: Keller & Partner Patentanwälte AG
(86) Internationale Anmeldenummer: PCT/CH2008/000327
(87) Internationale Veröffentlichungsnummer: WO 2009/055941

(56) Entgegenhaltungen:
- EP-A- 0 255 369
- EP-A- 0 528 294
- EP-A1- 0 254 701
- WO-A2-95/24237
- US-A- 6 096 055
- US-A1- 2002 038 103
- US-A1- 2005 288 632

## Beschreibung

### Technisches Gebiet

Die Erfindung betrifft einen Katheter zur Einführung in ein menschliches oder tierisches Hohlorgan, umfassend eine nach vorne ragende Katheterspitze, wobei hinter der Katheterspitze ein von einem expandierbaren röhrenförmigen Hohlkörper ummantelter Bereich angeordnet ist. Zudem bezieht sich die Erfindung auf einen Katheter zur Einführung in ein menschliches oder tierisches Hohlorgan, umfassend eine nach vorne ragende Katheterspitze und einen Hohlschaft. Des Weiteren betrifft die Erfindung Verfahren zur Herstellung eines Katheters.

### Stand der Technik

Katheter sind im Wesentlichen dünne Röhrchen oder Schläuche, welche zu therapeutischen oder diagnostischen Zwecken in menschliche oder tierische Hohlorgane oder Gefässe eingeführt werden. Insbesondere Harnröhren, Speiseröhren, Gallengänge oder blutführende Adern von Mensch und/oder Tier können mit Kathetern sondiert, durchstossen, entleert, gefüllt und/oder gespült werden. Die Einführbarkeit eines Katheters hängt dabei im Wesentlichen vom Aussendurchmesser bzw. der Querschnittsfläche des Katheters in der Einführrichtung ab. Ebenfalls entscheidend für eine gute Einführbarkeit ist zudem eine gute Flexibilität des Katheters, wobei jedoch zu beachten ist, dass eine gewisse Steifheit insbesondere im Bereich der Katheterspitze durchaus erwünscht ist, um den Katheter auch durch Verengungen im Hohlorgan oder im Gefäss hindurch zu bewegen.

Problematisch bei der Einführung sind insbesondere Katheter, welche zusätzlich expandierbare röhrenförmige Hohlkörper, wie beispielsweise Ballone oder medizinische Implantate, aufweisen. Derartige Hohlkörper können zwar relativ dicht zusammengefaltet und Platz sparend um den Katheter herum angeordnet werden. Dennoch ergeben sich aufgrund der Wandstärke der Hohlkörper und der übrigen Katheterelemente relativ grosse Aussendurchmesser der Katheter.

Aufgrund der mechanischen Anforderungen an die Katheterschäfte ist es jedoch nicht möglich, diese im Durchmesser beliebig zu verkleinern, und so den Aussendurchmesser des Katheters zu minimieren. Werden die Katheterschäfte nämlich zu klein dimensioniert, leidet die Einführbarkeit aufgrund der mangelnden Steifheit ebenso. Katheters sind bekannt aus US 2002/0038103, EP 0528294, US 6096055, US 2005/0288632, EP 0255369, EP 0254701 und WO 95/24237.

Es besteht daher nach wie vor ein Bedarf nach Kathetern mit expandierbaren röhrenförmigen Hohlkörpern, welche eine verbesserte Einführbarkeit aufweisen.

### Darstellung der Erfindung

Aufgabe der Erfindung ist es daher, einen dem eingangs genannten technischen Gebiet zugehörenden Katheter zu schaffen, welcher eine verbesserte Einführbarkeit in menschliche oder tierische Hohlorgane aufweist.

Die Lösung der Aufgabe ist durch die Merkmale des Anspruchs 1 definiert. Gemäss der Erfindung ist im ummantelten Bereich eine Drahtwendel angeordnet.

Unter einer Drahtwendel wird in diesem Zusammenhang ein hohlzylindrisches Gebilde verstanden, welches durch einen schraubenförmig um eine Längsachse gewundenen Draht gebildet wird.

Es hat sich überraschenderweise gezeigt, dass durch eine Drahtwendel innerhalb des ummantelten Bereichs der Aussendurchmesser des Katheters verringert werden kann, ohne die Knickstabilität des Katheters zu beeinträchtigen. Da die Drahtwendel verglichen mit einem Rohr bzw. einem Röhrchen gleichen Durchmesser in Längsrichtung als auch in Querrichtung eine hohe Flexibilität aufweist, beeinträchtig die Drahtwendel die Biegbarkeit des Katheters nur unwesentlich. Zusammen mit dem expandierbaren röhrenförmigen Hohlkörper ist aber in jedem Fall eine ausreichend grosse Steifheit vorhanden, welche eine Einführung des Katheters auch durch stark verengte Stellen in einem Hohlorgan ermöglicht. Eine Drahtwendel hat zudem den Vorteil, dass innwendig ein Freiraum vorliegt, in welchem weitere Bestandteile des Katheters, wie beispielsweise röntgendichte Marker oder Fluid führende Schäfte angeordnet werden können.

Zur Herstellung der erfindungsgemässen Katheter muss lediglich eine Drahtwendel mit einem expandierbaren röhrenförmigen Hohlkörper umgeben werden.

Bevorzugt ist eine Längsachse der Drahtwendel koaxial zu einer Längsachse des Katheters angeordnet. Eine derartige Anordnung ermöglicht einen möglichst Platz sparenden Einbau. Liegt der röhrenförmige Hohlkörper zudem direkt auf der Drahtwendel auf und ist an diese angeformt, wird die Gefahr des Verrutschens des röhrenförmigen Hohlkörpers während der Einführung des Katheters stark reduziert. Die Windungen der Drahtwendel, welche eine rippenartige Oberfläche bilden, ergeben in diesem Fall nämlich eine hohe Reibkraft zwischen der Drahtwendel und dem röhrenförmigen Hohlkörper, welche in Längsrichtung des Katheters wirkt.

Zudem hat es sich als vorteilhaft erwiesen, wenn sich die Drahtwendel in einer Längsrichtung, welche parallel zur Längsachse des Katheters verläuft, von einem vorderen Ende des expandierbaren röhrenförmigen Hohlkörpers bis an ein hinteres Ende des expandierbaren röhrenförmigen Hohlkörpers erstreckt. Ist die Drahtwendel beispielsweise als Halter für eine röntgendichte Markierung vorgesehen, kann somit im Prinzip jede beliebige Position des expandierbaren röhrenförmigen Hohlkörpers, wie insbesondere das vordere Ende und/oder das hintere Ende, markiert werden. Auch im Hinblick auf das Verrutschen eines direkt auf der Drahtwendel aufliegenden röhrenförmigen Hohlkörpers hat diese Anordnung Vorteile, da die rippenartige Oberfläche der Drahtwendel nämlich auf der gesamten Länge des expandierbaren röhrenförmigen Hohlkörpers vorliegt.

Es ist aber auch möglich, die Drahtwendel kürzer auszugestalten, so dass lediglich in einem Teilbereich des vom expandierbaren röhrenförmigen Hohlkörper ummantelten Bereichs eine Drahtwendel vorliegt. Diese kann beispielsweise im Bereich des vorderen Endes, aber auch im Bereich des hinteren Endes des expandierbaren röhrenförmigen Hohlkörpers sein.

Es hat sich zudem als vorteilhaft erwiesen, die Drahtwendel innerhalb eines im ummantelten Bereich angeordneten Hohlschafts des Katheters anzuordnen, wobei es sich bevorzugt um einen innersten Hohlschaft des Katheters handelt. Dabei ist eine Längsachse des Hohlschafts insbesondere koaxial zur Längsachse der Drahtwendel angeordnet. Damit ist sichergestellt, dass die Drahtwendel nicht als aussen angeordnete Vorrichtung zu einer Vergrösserung des Katheterdurchmessers führt. Zudem werden eventuell vorhandene scharfkantige Vorstände der Drahtwendel durch den Hohlschaft bestmöglich abgedeckt, so dass auch mechanisch empfindliche expandierbare röhrenförmige Hohlkörper, wie z. B. Ballone, am Katheter angebracht werden können.

Es ist aber grundsätzlich auch möglich, die Drahtwendel ausserhalb eines Hohlschafts anzuordnen, falls dies aus anderen Gründen zweckdienlich ist. Zudem kann die Drahtwendel auch nur teilweise, z. B. mit ihrem hinteren Ende, in einem Hohlschaft angeordnet werden. In diesem Fall kann das vordere Ende der Drahtwendel aus dem Hohlschaft herausragen und beispielsweise direkt vom expandierbaren röhrenförmigen Hohlkörper umgeben sein. Es liegt aber auch im Rahmen der Erfindung, das hintere Ende der Drahtwendel in einem äusseren Hohlschaft anzuordnen, während das vordere Ende des Hohlschafts aussen auf einem Innenschaft des Katheters aufliegt.

Ist die Drahtwendel innerhalb eines Hohlschafts angeordnet, so wird der Hohlschaft dabei besonders bevorzugt derart an die Drahtwendel angeformt, dass eine äussere Oberfläche des Hohlschafts im Wesentlichen einer äusseren Kontur der Drahtwendel entspricht. Damit liegt auch im Bereich der äusseren Oberfläche des Hohlschafts eine rippenartige Oberfläche vor, was, wie vorstehend bereits ausgeführt, insbesondere die Gefahr des Verrutschens des expandierbaren röhrenförmigen Hohlkörpers verringert. Grundsätzlich ist es beispielsweise auch möglich, zusätzlich zur Anformung oder anstelle der Anformung eine Prägestruktur oder vorstehende Rippen an der äusseren Oberfläche des Hohlschafts anzubringen, welche insbesondere quer zu einer Längsrichtung des Hohlschafts verlaufen.

Bei der Herstellung eines Katheters wird die Drahtwendel vor einem Anbringen des expandierbaren röhrenförmigen Hohlkörpers bevorzugt in einen Hohlschaft des Katheters eingeführt und anschliessend wird der Hohlschaft an eine äussere Kontur der Drahtwendel angeformt. Dies kann beispielsweise durch eine Erwärmung des Hohlschafts erfolgen. Es kann aber auch ausreichen, eine relativ satt sitzende Drahtwendel in den Hohlschaft einzuführen. Da die bei Kathetern üblicherweise verwendeten Hohlschäfte meist aus weichem und flexiblen Material bestehen und zudem dünnwandig sind, können sich derartige Hohlschäfte auch selbsttätig an die Kontur der Drahtwendel anpassen.

Die Drahtwendel weist mit Vorteil einen Aussendurchmesser von 0.2-0.5 mm und einen Drahtdurchmesser von 30-100 µm auf. Es hat sich gezeigt, dass derartige Drahtwendel die erforderliche Flexibilität, gleichzeitig aber noch eine ausreichende Steifheit aufweisen. Der Aussendurchmesser von 0.2-0.5 mm entspricht zudem den heute üblicherweise verwendeten Innendurchmessern von Katheterschäften. Die Drahtwendel könne damit in bereits auf dem Markt erhältlichen Katheterschäften integriert werden, was hilft die Produktionskosten der Erfindungsgemässen Katheter gering zu halten. Im Prinzip können aber auch anders dimensionierte Drahtwendel verwendet werden. Weisen diese aber beispielsweise einen zu grossen Drahtdurchmesser auf, wird die Flexibilität des Katheters verringert.

Insbesondere kann innerhalb der Drahtwendel ein geradliniger Innendraht angebracht sein, welcher in einem oder mehreren Verbindungsbereichen punktuell mit der Drahtwendel verbunden ist. Dadurch kann beispielsweise die Flexibilität und/oder die Steifheit einer Drahtwendel modifiziert werden. Drahtwendel mit einem kleinen Aussendurchmesser, welche eine hohe Flexibilität aufweisen, können so beispielsweise steifer gemacht werden. Die Verbindung zwischen der Drahtwendel und dem Innendraht kann beispielsweise durch eine stoffschlüssige Verbindungstechnik, insbesondere durch Verlötung oder durch Verschweissung erfolgen.

Bevorzugt ist die Drahtwendel und der geradlinige Innendraht in den Verbindungsbereichen mit einem röntgendichten Lot, insbesondere einem Silberlot, verlötet. Damit können in einfacher Art und Weise röntgendichte Bereiche des Katheters gebildet werden. Röntgendichte Bereiche sind insbesondere wichtig, um die exakte Position des Katheters im Hohlorgan zu überprüfen. Unter "röntgendichte Bereiche" wird in diesem Zusammenhang und nachfolgend verstanden, dass die röntgendichten Bereiche in einem Bildgebungsverfahren am menschlichen und/oder tierischen Körper aufgrund der Absorption von eingestrahlter Röntgenstrahlung von umliegenden Bereichen des Katheters und/oder vom umliegenden Gewebe unterscheidbar sind, wobei die Röntgenstrahlung in einer üblichen Dosis eingestrahlt wird, welche für den menschlichen und/oder tierischen Körper unbedenklich ist. Auf die sonst üblicherweise angebrachten und sperrigen röntgendichten Ringe, welche um einzelne Schäfte des Katheters herum angeordnet sind, kann somit verzichtet werden, was hilft die Abmessungen des Katheters gering zu halten. Das vorteilhaft verwendete Silberlot ist für den menschlichen und/oder tierischen Körper zudem unbedenklich, so dass von diesem Material keine Gefahr für den Patienten oder das Tier ausgeht.

Besonders vorteilhaft besteht die Drahtwendel selbst aus röntgendichtem Material, insbesondere aus Platin, Gold oder Wolfram. Röntgendichte Materialien zeichnen sich dabei durch einen grossen Absorptionsquerschnitt für Röntgenstrahlung aus. Rostfreier Stahl, welcher gegenüber Platin, Gold oder Wolfram einen weitaus kleineren Absorptionsquerschnitt aufweist, kann unter den üblichen Bedingungen bei der Bildgebung mit Röntgenstrahlung am menschlichen und/oder tierischen Körper nicht abgebildet werden und ist daher nicht röntgendicht bzw. röntgendurchlässig. Wird die Drahtwendel selbst aus röntgendichtem Material hergestellt, so kann zur Bildung von röntgendichten Bereichen am Katheter bei einer ausreichenden Länge des Drahtwendels ebenfalls auf röntgendichte Ringe verzichtet werden. Da damit grundsätzlich überhaupt kein zusätzliches röntgendichtes Material am Katheter mehr angeordnet werden muss, ermöglicht dies einen besonders einfachen Katheteraufbau.

Es ist aber beispielsweise auch möglich, in einem vorderen Bereich des expandierbaren röhrenförmigen Hohlkörpers eine Drahtwendel aus röntgendichtem Material anzuordnen, und in einem hinteren Bereich einen röntgendichten Markierungsring und/oder ein röntgendichtes Lot anzubringen. Damit wird die Einführbarkeit des Katheters bereits erheblich verbessert, da zumindest der vordere Bereich des Katheters einen geringeren Durchmesser aufweisen kann.

Bevorzugt sind mehrere benachbarte Windungen der Drahtwendel aneinander anliegend angeordnet und bilden so einen röntgendichten Bereich des Katheters. Es hat sich nämlich gezeigt, dass zur Bildung eines röntgendichten Bereichs wenigstens fünf benachbarte Windungen der Drahtwendel aus röntgendichtem Material aneinander anliegen sollten, um einen optimalen Kontrast beim bildgebenden Verfahren mit Röntgenstrahlung am menschlichen und/oder tierischen Körper zu erhalten. Es ist auch möglich, die Windungen nicht direkt aneinander anliegend anzuordnen oder weniger als fünf aneinander liegende Windungen als röntgendichten Bereich vorzusehen. Der Kontrast im bildgebenden Verfahren nimmt aber entsprechend ab.

Es hat sich gezeigt, dass Bereiche der Drahtwendel, bei welchen zwischen den einzelnen Windungen ein Abstand vorliegt, welcher wenigstens dem zweifachen, insbesondere dem dreifachen Drahtdurchmesser entspricht, beim bildgebenden Verfahren mit Röntgenstrahlung am menschlichen und/oder tierischen Körper nicht mehr sichtbar sind. Als Abstand ist dabei der eigentliche Freiraum zwischen den Windungen massgebend. Unter einer Windung wird in diesem Zusammenhang ein Bereich der Drahtwendel verstanden, weicher die Längsachse der Drahtwendel genau einmal umläuft.

Zur Erzeugung eines röntgendurchlässigen Bereichs der Drahtwendel aus röntgendichtem Material sind daher bevorzugt benachbarte Windungen berührungslos und voneinander beabstandet angeordnet, so dass zwischen den einzelnen Windungen insbesondere ein Abstand vorliegt, welcher wenigstens dem zweifachen, bevorzugt dem dreifachen Drahtdurchmesser entspricht.

Damit können gezielt einzelne Bereich des Katheters röntgendicht markiert werden, während andere Bereiche röntgendurchlässig ausgebildet sind. Es ist aber auch möglich, einen Drahtwendel aus röntgendichtem Material zu verwenden, welcher über seine gesamte Länge über aneinander anliegende Windungen verfügt und daher vollständig röntgendicht ist.

Insbesondere können zur Längenmarkierung mehrere röntgendichte Bereiche und mehrere röntgendurchlässige Bereiche der Drahtwendel in einer alternierenden Abfolge und insbesondere in regelmässigen Abständen angeordnet sein. Die im Röntgenbild sichtbaren Längenmarkierungen liefern dem Arzt beispielsweise Informationen zur Lage des Katheters relativ zur Bildebene des Röntgenbilds. Liegen die Längenmarkierungen beispielsweise eng zusammen, verläuft der Katheter in einer Richtung aus der Bildebene heraus. Zeigen die Längenmarkierungen den Maximalabstand, so liegt der Katheter parallel zur Bildebene. Wird die Lage des Katheters aus unterschiedlichen Richtungen abgebildet, ist es somit grundsätzlich möglich, die räumliche Lage des Katheters zu bestimmen. Die Längenmarkierungen müssen nicht zwingend im ummantelten Bereich des Katheters vorliegen. Es ist auch möglich, diese in einem Bereich der Drahtwendel anzuordnen, welcher aus dem ummantelten Bereich nach hinten ragt.

Bei der Herstellung eines Katheters mit einer derartigen Drahtwendel wird diese vor einem Anbringen des expandierbaren röhrenförmigen Hohlkörpes in einem ersten Bereich gestaucht und in einem zweiten Bereich, welcher direkt an den ersten Bereich angrenzt, gestreckt. Dies wird so oft wie notwenig wiederholt, bis eine gewünschte Abfolge von röntgendichten und röntgendurchlässigen Bereichen vorliegt.

Die erfindungsgemässe Lösung eignet sich insbesondere, wenn wenigstens zwei expandierbare röhrenförmige Hohlkörper vorliegen, welche koaxial übereinander liegend angeordnet sind. Derartige Katheter weisen im Bereich der beiden expandierbaren röhrenförmigen Hohlkörper meist einen relativ grossen Aussendurchmesser auf. Schon geringe Einsparungen im Aussendurchmesser helfen hier, die Einführbarkeit markant zu verbessern, da die Querschnittsfläche quadratisch mit dem Durchmesser bzw. mit dem Radius zunimmt. Durch die erfindungsgemässe Drahtwendel lässt sich der Aussendurchmesser bzw. die Querschnittsfläche des Katheters so gering als möglich halten. Gleichzeitig ist aber eine gute Stabilität und Flexibilität gewährleistet, wodurch die Einführbarkeit des Katheters verbessert wird. Es ist aber auch möglich, lediglich einen einzelnen expandierbaren röhrenförmigen Hohlkörper vorzusehen.

Insbesondere ist als expandierbarer röhrenförmiger Hohlkörper ein beaufschlagbarer Ballon angeordnet. Der Ballon kann im Prinzip um die Drahtwendel herum in zusammengefalteter Form angeordnet sein. Die Drahtwendel ermöglicht in diesem Fall eine besonders effektive Beaufschlagung des Ballons. Ein Fluid kann nämlich durch die Freiräume zwischen den einzelnen Windungen der Drahtwendel direkt in den zusammengefalteten Ballon geleitet werden. Die Beaufschlagung erfolgt dann entlang der gesamten Länge der Drahtwendel, was eine gleichmässige und rasche Beaufschlagung des Ballons ermöglicht. Es ist aber auch möglich, den Ballon um eine in einem Schaft angeordnete Drahtwendel herum anzuordnen. Die Beaufschlagung kann dann beispielsweise durch spezielle Öffnungen in der Mantelfläche des Hohlschafts erfolgen. Die im Innern des Hohlschafts angeordnete Drahtwendel stellt für das im Hohlschaft geleitete Fluid dabei keinen grossen Widerstand dar, so dass auch in diesem Fall eine Beaufschlagung des Ballons erfolgen kann. Ist der Hohlschaft zusätzlich an eine Kontur der Drahtwendel angeformt, kann das während der Beaufschlagung zwischen den Hohlschaft und den zusammengefalteten Ballon eintretende Fluid insbesondere entlang des Hohlschafts besser verteilt werden. Aufgrund der unebenen Oberfläche des Hohlschafts kann sich das Fluid nämlich besser ausbreiten, was zu einer gleichmässigeren Beaufschlagung des Ballons führt. Unter einer gleichmässigen Beaufschlagung wird verstanden, dass sich der Ballon entlang seiner gesamten Länge gleichmässig aufweitet. Dies ist insbesondere dann wichtig, wenn der Ballon beispielsweise im Bereich einer Verengung in einem Hohlorgan vorliegt, welcher aufgeweitet werden soll. Befindet sich dabei ein Teil des Ballons hinter oder vor der Verengung, so kann es bei einer ungleichmässigen Beaufschlagung vorkommen, dass sich die Position des Katheters im Hohlorgan verschiebt und dieser aus dem Bereich der Verengung herausrutscht. Bei einer gleichmässigen Beaufschlagung ist diese Gefahr weitaus kleiner, da der in der Verengung befindliche Teil des Ballons von Anfang an mit beaufschlagt wird und somit der Ballon in der Verengung verklemmt.

Wie vorstehend beschrieben ist es nun möglich, einen röntgendichten Bereich direkt in der Drahtwendel zu realisieren. Es kann beispielsweise röntgendichtes Silbelot in die Drahtwendel eingebracht werden oder die Drahtwendel wird direkt aus röntgendichtem Material gefertigt. In beiden Fällen führt der röntgendichte Bereich jedoch nicht zu einer Zunahme des Aussendurchmessers des Katheters.

Insbesondere kann der Ballon eine Beschichtung aufweisen, wobei die Beschichtung wenigstens ein Arzneimittel enthält. Die Beschichtung kann dann in einem zu behandelnden Bereich an die Wände des Hohlorgans gedrückt werden, so dass das Arzneimittel vom Hohlorgan aufgenommen werden kann. Da derartige Beschichtungen ebenfalls eine signifikante Schichtdicke aufweisen, ist es auch in diesem Fall von grossem Vorteil, den Katheter mit anliegendem Ballon im Durchmesser so klein als möglich auszugestalten.

Als expandierbarer röhrenförmiger Hohlkörper kann beispielsweise auch ein medizinisches Implantat, insbesondere ein Stent, angeordnet sein, welche bevorzugt an eine äussere Kontur der Drahtwendel angeformt ist. Liegt das medizinische Implantat als ein einziger expandierbarer röhrenförmiger Hohlkörper direkt auf der Drahtwendel und/oder auf einem Hohlschaft des Katheters, welcher an die Kontur der Drahtwendel angeformt ist, so bringt dies ebenfalls grosse Vorteile mit sich. Die unebene Struktur der Drahtwendel oder/des angeformten Hohlschafts stellt nämlich sicher, dass das medizinische Implantat während dem Einführen des Katheters in das Hohlorgan nicht in axialer Richtung nach vorne oder nach hinten verschoben oder gar vom Katheter abgestreift wird.

Auch ein medizinisches Implantat kann eine Beschichtung aufweisen, wobei die Beschichtung wenigstens ein Arzneimittel enthält. Wie beim Katheter mit Ballon ermöglicht die erfindungsgemässe Drahtwendel auch bei einem medizinischen Implantat eine äusserst kompakte Bauweise des Katheters, was die Einführbarkeit stark verbessert.

Bevorzugt ist die Katheterspitze als flexible Schraubenfeder ausgebildet. Dabei ist es möglich, einen durchgehenden Drahtwendel anzuordnen, welcher von der Spitze des Katheters bis in den ummantelten Bereich hinein und gegebenenfalls am hinteren Ende wieder aus diesem heraus reicht. Dies vereinfacht insbesondere die Produktion der erfindungsgemässen Katheter. Es ist aber auch möglich, eine anders ausgeformte Spitze vorzusehen, welche z. B. im Hinblick auf eine bestimmte Anwendung optimiert ist.

Bei einem Katheter zur Einführung in ein menschliches oder tierisches Hohlorgan, umfassend eine nach vorne ragende Katheterspitze und einen Hohlschaft, ist es auch möglich, auf dem Hohlschaft einen röntgendichten Ring, insbesondere aus Platin, Gold oder Wolfram, anzuordnen und am Hohlschaft in einem Bereich vor und/oder hinter dem röntgendichten Ring mehrere den Hohlschaft umlaufende und von diesem abstehende rippenartige Vorstände anzubringen. Ein derartiger Katheter weist ebenfalls eine verbesserte Einführbarkeit auf. Zur Herstellung wird dabei bevorzugt in einem ersten Verfahrensschritt ein röntgendichter Ring auf den Hohlschaft aufgebracht. Zur Bildung von rippenartigen Vorständen wird der Hohlschaft anschliessend in mehreren Bereichen vor dem röntgendichten Ring und/oder in mehreren Bereichen hinter dem röntgendichten Ring in einer Längsrichtung des Hohlschafts gestaucht.

Es hat sich gezeigt, dass derartige rippenartige Vorstände insbesondere die Dehnfähigkeit bzw. die Flexibilität des Hohlschafts verbessern. Zudem wirken die vom Hohlschaft abstehenden rippenartigen Vorstände als Kantenschutz für den röntgendichten Ring. Dies ist insbesondere bei Kathetern mit Ballonen von grossem Vorteil, da die gegenüber mechanischen Einwirkungen meist sehr empfindlichen Ballonhüllen bestmöglich vor einer Beschädigung geschützt werden. Des Weiteren wird der röntgendichte Ring gleichzeitig in axialer Richtung fixiert. Eine Verschiebung des Rings beim Einführen des Katheters in ein Hohlorgan ist so praktisch ausgeschlossen.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Einen Querschnitt durch einen Katheter mit einer Drahtwendel in einem Hohlschaft und einem darum herum angeordneten und zusammen gefalteten Ballon, welcher von einem Stent umgeben ist;
- Fig. 2: einen Querschnitt durch einen Katheter mit einer Drahtwendel, welche direkt von einem Stent umgeben ist;
- Fig. 3: einen Querschnitt durch einen Katheter mit einer Drahtwendel in einem vorderen Bereich eines Hohlschafts und einem röntgendichten Markierungsring auf dem Hohlschaft, wobei der Hohlschaft vor und nach dem Markierungsring rippenartige Vorstände aufweist.

Grundsätzlich sind in den Figuren gleiche Teile mit gleichen Bezugszeichen versehen.

### Wege zur Ausführung der Erfindung

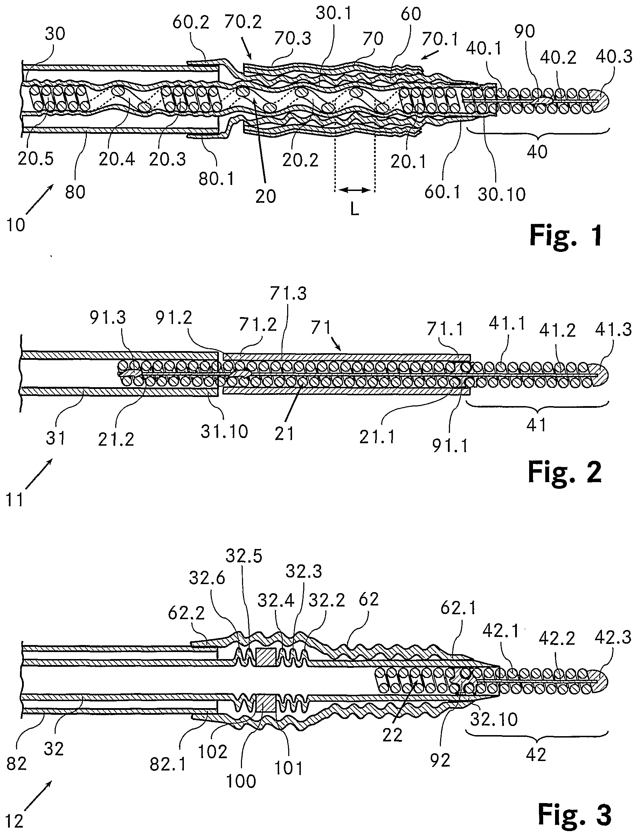

In Fig. 1 ist ein erster erfindungsgemässer Katheter 10 im Querschnitt abgebildet. Der Katheter 10 verfügt über einen Aussenschaft 80, aus welchem in Längsrichtung ein Hohlschaft 30 nach rechts herausragt. Am vorderen Ende 30.10 des Hohlschafts 30 ist das hintere Ende einer Katheterspitze 40 im Hohlschaft 30 verankert, so dass die Katheterspitze 40 in Längsrichtung aus dem Hohlschaft 30 herausragt. Die Katheterspitze 40 besteht aus einer zylindrischen Schraubenfeder 40.1, welche im Zentrum einen Stützdraht 40.2 aufweist, welcher zur Stabilisierung entlang der gesamten Länge der Schraubenfeder 40.1 verläuft. Der Stützdraht 40.2 ist dabei ungefähr in der Mitte in Längsrichtung der Schraubenfeder 40.1 durch eine Lotstelle 90 aus Silberlot stoffschlüssig mit der Schraubenfeder 40.1 verbunden. Die Schraubenfeder 40 weist am vorderen Ende zudem eine abgerundete Endkappe 40.3 auf. Zur Verankerung der Schraubenfeder 40 sind die hintersten und im Innern des Hohlschafts 30 angeordneten Windungen der Schraubenfeder 40.1 im vorderen Ende 30.10 des Hohlschafts 30 eingepresst.

In einem Bereich hinter der Schraubenfeder 40.1 ist zudem eine Drahtwendel 20 aus Platin koaxial im Hohlschaft 30 angeordnet. Die Drahtwendel weist z. B. einen Aussendurchmesser von 0.4 mm auf, wobei die Drahtdicke der Drahtwendel 20 beispielsweise 50 µm misst. Die Drahtwendel 20 weist in einem vordersten bzw. ersten Bereich 20.1 fünf aneinander liegende Windungen auf. Der erste Bereich 20.1 der Schraubenfeder 20 ist aufgrund des Materials (Platin) und der Dichte an Windungen röntgendicht. In einem an den ersten Bereich 20.1 anschliessenden zweiten Bereich 20.2 verfügt die Drahtwendel 20 über drei vollständig berührungslos angebrachte Windungen, wobei zwischen zwei Windungen in Längsrichtung ein Abstand L von ca. 160 µm vorliegt. Der Abstand L entspricht in etwa der dreifachen Drahtdicke der Drahtwendel 20. Aufgrund der geringen Dichte an Windungen ist der zweite Bereich 20.2 der Schraubenfeder 20 röntgendurchlässig. An den zweiten Bereich 20.2 der Drahtwendel 20 schliesst der dritte Bereich 20.3 an, welcher wieder über fünf aneinander liegende Windungen der Schraubenfeder 20 verfügt und daher ebenfalls röntgendicht ist. Der hinter dem dritten Bereich 20.3 liegende vierte Bereich 20.4 der Schraubenfeder 20 verfügt über eine berührungslos angeordnete Windung, wodurch dieser Bereich rötgendurchlässig ist. An den vierten Bereich 20.4 schliesst der fünfte Bereich 20.5 der Schraubenfeder an, welcher im Wesentlichen baugleich ist mit dem dritten Bereich 20.3 der Schraubenfeder 20. Hinter dem fünften Bereich 20.5 sind weitere in Fig. 1 nicht dargestellte Bereiche der Schraubenfeder 20 angeordnet. Dabei wechseln sich Bereiche mit berührungslos angeordneten Windungen (analog dem vierten Bereich 20.4) mit Bereichen mit aneinander liegenden Windungen (analog zum dritten Bereich 20.3) ab und bilden ein regelmässiges Muster aus röntgendichten und röntgendurchlässigen Bereichen.

Der Hohlschaft 30 ist durchwegs an die äussere Form bzw. Kontur der Drahtwendel 20 angeformt. Die äussere Oberfläche 30.1 des Hohlschafts 30 weist daher eine schraubenförmige Struktur auf, wobei sich Bereich mit geringer Gewindesteigung und Bereich mit grosser Gewindesteigung abwechseln.

Am vorderen Ende 80.1 des Aussenschafts 80 ist entlang des gesamten Umfangs des Aussenschafts 80 zudem das hintere Ende 60.2 eines beaufschlagbaren und expandierbaren Ballons 60 angebracht. Der Ballon 60 ist dabei als röhrenförmiger Hohlkörper ausgebildet und mit seiner Längsachse koaxial zur Längsachse des Katheters 10 angeordnet. Der vor dem Aussenschaft 80 liegende Teil des Ballons 60 liegt dabei direkt auf dem schraubenartig strukturierten Hohlschaft 30 auf und ist mit seinem vorderen Ende 60.1 im Bereich des vorderen Endes 30.10 des Holschafts 30 mit diesem verschweisst. Die ersten beiden Bereiche 20.1, 20.2 der Drahtwendel sind dabei vollständig vom Ballon 60 ummantelt.

Ausserhalb des Ballons 60 ist des Weiteren ein durch den Ballon 60 expandierbarer Stent 70 bzw. ein röhrenförmiges medizinisches Implantat ebenfalls koaxial zur Längsachse des Katheters angebracht. Der Stent 70 ist dabei von aussen an den Ballon 60 angepresst und an die äussere Oberfläche 30.1 des Hohlschafts 30 bzw. an die Kontur der Drahtwendel 20 im in den ersten beiden Bereichen 20.1, 20.2 angeformt. Das hintere Ende 70.2 des Stents 70 liegt dabei vor dem hinteren Ende 60.2 des Ballons 60. Gleichzeitig liegt das vordere Ende 70.1 des Stents 70 hinter dem vorderen Ende 60.1 des Ballons 60.

Im Bereich der äusseren Mantelfläche ist der Stent 70 zudem mit einer Beschichtung 70.3, welche ein Arzneimittel enthält, umgeben.

Bei einer Beaufschlagung des Ballons 60 kann sich das dabei verwendete Fluid aufgrund der schraubenförmigen Struktur der äusseren Oberfläche 30.1 des Hohlschafts 30 entlang der gesamten Länge des Ballons 60 ausdehnen, wodurch eine gleichmässige Beaufschlagung des Ballons 60 resultiert. Damit wird auch die Gefahr vermindert, dass der Stent 70 während der Beaufschalgung des Ballons 60 in Längsrichtung des Katheters 10 verrutschen oder gar von diesem abrutschen kann.

Fig. 2 zeigt einen zweiten erfindungsgemässen Katheter 11 im Querschnitt. Der Katheter 11 weist einen Holschaft 31 auf, in welchem in einem Bereich des vorderen Endes 31.10 koaxial eine Drahtwendel 21 aus rostfreiem Stahl angeordnet ist, welche aus dem Hohlschaft 31 herausragt. Die Drahtwendel 21 weist einen Aussendurchmesser von beispielsweise 0.5 mm und einen Drahtdurchmesser von z. B. 100 µm auf. Im Innern der Drahtwendel 21 ist ein geradliniger Stützdraht 41.2 angebracht. Im Bereich des vorderen Endes 21.1 der Drahtwendel 21 ist diese mit einer ersten Lotstelle 91.1 aus Silberlot mit einer Katheterspitze 41 verlötet. Die Katheterspitze 41 besteht aus einer Schraubenfeder 41.1 aus rostfreiem Stahl und weist am vorderen Ende eine abgerundete Endkappe 41.3 auf.

Im Innern der Schraubenfeder 41.1 ist ein Stützdraht 41.2 angeordnet, welcher von der Endkappe 41.3 der Katheterspitze 41 her durch die erste Lotstelle 91.1 und die Drahtwendel bis an deren hinteres Ende 21.2 ragt. Kurz vor dem vorderen Ende 31.10 des Hohlschafts 31 liegt eine zweite Lotstelle aus Silberlot vor, welche den Stützdraht 41.2 und die Drahtwendel miteinander verbindet. Am hinteren Ende der Drahtwendel liegt eine dritte Lotstelle vor, welche das hintere Ende des Stützdrahts 41.2 mit der Drahtwendel 21 verbindet.

Direkt vor dem vorderen Ende 31.10 des Hohlschafts 31 ist ein zylindrischer Stent 71 bzw. ein röhrenförmiges medizinisches Implantat um die Drahtwendel 21 herum angeordnet. Das hintere Ende 71.2 des Stents 71 umgibt die zweite Lotstelle 91.2, während die erste Lotstelle 91.1 vom vorderen Ende 71.1 des Stents 71 umgeben ist. An den drei Lotstellen 91.1, 91.2, 91.3 liegt dabei ausreichend Silberlot vor, so dass diese röntgendicht sind.

Der Stent 71 ist zudem an die Drahtwendel 21 angepresst, so dass in Fig. 2 nicht dargestellte Unebenheiten der inneren Oberfläche 71.3 des röhrenförmigen Stents 71 in die Zwischenräume zwischen den einzelnen Windungen der Drahtwendel 21 gedrückt werden. Damit wird eine Verschiebung des Stents 71 in einer Längsrichtung des Katheters 11 stark erschwert, was insbesondere beim Einführen des Katheters 11 vorteilhaft ist, da der Stent 71 so kaum vom Katheter 11 abrutschen kann.

Der Stent 71 ist als selbst-expandierbarer Hohlkörper ausgebildet und wird durch eine nicht dargestellte Umhüllung in der in Fig. 2 dargestellten komprimierten Form gehalten.

Ein dritter erfindungsgemässer Katheter 12 ist in Fig. 3 dargestellt. In einem Aussenschaft 82 verläuft dabei ein Hohlschaft 32 und ragt aus dem vorderen Ende 82.1 des Aussenschafts 82 nach vorne. In einem Bereich vor dem vorderen Ende 82.1 des Aussenschafts 82 ist ein röntgendichter Ring 100, beispielsweise aus Platin, um den Hohlschaft 32 herum angeordnet. Der Hohlschaft 32 weist in einem Bereich direkt vor dem röntgendichten Ring 100 drei hintereinander angeordnete und den Hohlschaft 32 vollständig umlaufende Rippen 32.2, 32.3, 32.4 auf. Die drei Rippen 32.2, 32.3, 32.4 ragen dabei von einer Mantelfläche des Hohlschafts 32 nach aussen und bestehen aus aufgestauchtem Material des Hohlschafts 32, welcher aus Kunststoff gefertigt ist. In gleicher Weise sind hinter dem röntgendichten Ring 100 zwei weitere Rippen 32.5, 32.6 angeordnet. Die fünf Rippen 32.2...32.6 des Hohlschafts 32 weisen dabei einen Aussendurchmesser auf, welcher dem Aussendurchmesser des röntgendichten Rings 100 entspricht. Dadurch ist der röntgendichte Ring 100 zwischen den vorderen drei Rippen 32.2, 32.3, 32.4 und den hinteren beiden Rippen 32.5, 32.6 eingebettet. Insbesondere ist die hinter Kante 102 und die vordere Kante 101 des röntgendichten Rings 100 so in Längsrichtung des Katheters 11 von den Rippen 32.2...32.6 abgedeckt.

Im Bereich des vorderen Endes 32.10 des Hohlschafts 32 ist eine Schraubenfeder 42.1 aus rostfreiem Stahl als Bestandteil einer Katheterspitze 42 im Hohlschaft 32 verankert. Die Schraubenfeder 42.1 weist im Innern einen in Längsrichtung verlaufenden Stützdraht 42.2 und am vorderen Ende eine abgerundete Endkappe 42.3 auf.

Hinter der Schraubenfeder 42.1 ist im Hohlschaft 32 eine Drahtwendel 22 mit sechs Windungen angeordnet, welche über eine Lotstelle 92 mit dem hinteren Ende der Schraubenfeder 42.1 verbunden ist. Die Drahtwendel besteht aus Wolfram und weist einen Aussendurchmesser von beispielsweise 0.2 mm auf. Die Drahtdicke der Drahtwendel beträgt z. B. 30 µm. Aufgrund der aneinander liegenden Windungen der Drahtwendel 22 ist diese röntgendicht.

Ausserhalb des Hohlschafts 32 ist des Weiteren ein beaufschlagbarer Ballon 62 um den Hohlschaft 32 herum angeordnet. Das vordere Ende 62.1 des Ballons 62 ist dabei im Bereich des vorderen Endes 32.10 des Hohlschafts 32 mit diesem verschweisst. Das hintere Ende 62.2 des Ballons 62 ist am vorderen Ende 82.1 des Aussenschafts 82 aussenseitig angeschweisst.

Bei der Herstellung des Katheters 12 aus Fig. 3 wird in einem ersten Schritt ein röntgendichter Ring 100 auf einen Hohlschaft 32 geschoben. Anschliessend wird der Hohlschaft 32 beispielsweise im Bereich vor dem röntgendichten Ring 100 zur Bildung einer ersten Rippe 32.4 gestaucht und anschliessend wieder leicht gestreckt. Eine zweite Rippe 32.3 und eine dritte Rippe 32.2 werden in analoger Weise gebildet. Anschliessend werden die Rippen 32.5, 32.6 hinter dem röntgendichten Ring 100 in gleicher Weise erzeugt.

Die beschriebenen Ausführungsformen sind als illustrative Beispiele zu verstehen, welche im Rahmen der Erfindung beliebig erweitert oder abgewandelt werden können.

So ist es z. B. auch möglich, anstelle der drei beschriebenen Katheterspitzen 40, 41, 42 auch Katheterspitzen vorzusehen, welche anstelle der Schraubenfedern 40.1, 41.1, 42.1 über eine andere geeignete flexible Vorrichtung verfügen. Ebenso ist es möglich, die Schraubenfedern 40.1, 41.1, 42.1 der Katheterspitzen aus einem röntgendichten Material, wie z. B. Gold, Platin oder Wolfram zu fertigen, so dass die gesamte Katheterspitze röntgendicht ist. Eine weitere Möglichkeit besteht darin, die drei Drahtwendel 20, 21, 22 und die drei Schraubenfeder 40.1, 41.1, 42.1 aus einer einzigen Schraubenfeder, insbesondere aus röntgendichtem Material, auszubilden, welche entsprechend der Erfindung bis in die von den expandierbaren Hohlkörpern ummantelten Bereiche der Katheter reichen.

Bei dem in Fig. 1 beschriebenen ersten Katheter 10 kann die Anzahl der aneinander liegenden Windungen und/oder der berührungslos angeordneten Windungen der Drahtwendel 20 vergrössert werden, um beispielsweise eine bessere Sichtbarkeit beim Röntgen zu erhalten. Hierfür ist es auch möglich, in den röntgendichten Bereichen der Drahtwendel 20 zusätzlich röntgendichtes Silberlot anzubringen.

Die Drahtwendel 20 beim ersten Katheter 10 muss zudem nicht zwingend bis in den Bereich des Aussenschafts 80 hinein ragen. Es ist auch möglich, eine Drahtwendel vorzusehen, welche wie beim dritten Katheter 12 der Fig. 3 lediglich in einen vorderen Bereich des Ballons 60 bzw. des Stents 70 hinein ragt.

Es ist auch möglich, die Beschichtung 70.3 des Stents 70 beim ersten Katheter 10 wegzulassen oder durch eine andere Beschichtung zu ersetzen. Insbesondere geeignet sind beispielsweise Beschichtungen aus Silikon, welche die Gleiteigenschaften des Katheters verbessern.

Auch kann der Stent 70 beim ersten Katheter 10 weggelassen werden, so dass der Ballon 60 als äusserter expandierbarer Hohlkörper vorliegt. In diesem Fall kann z. B. der Ballon auch mit einer Beschichtung mit Arzneimitteln oder einer Beschichtung zur Verbesserung der Gleiteigenschaften versehen werden.

Beim zweiten Katheter 11 aus Fig. 2 kann die Drahtwendel 21 im Bereich des Stents 71 auch gestreckt werden bzw. so ausgebildet sein, dass die einzelnen Windungen der Drahtwendel berührungslos angeordnet sind. Dadurch ist es unter Umständen möglich, die Reibung zwischen dem Stent 71 und der Drahtwendel 21 noch weiter zu erhöhen. Anstelle einer Drahtwendel aus rostfreiem Stahl beim zweiten Katheter auch eine Drahtwendel aus röntgendichtem Material, wie Gold Platin, oder Wolfram, eingesetzt werden.

Zusätzlich zu dem in Fig. 3 gezeigten röntgendichten Ring 100 können zudem auch weitere röntgendichte Ringe an einem Schaft des Katheters angeordnet sein.

Zusammenfassend ist festzustellen, dass ein Katheter mit expandierbarer Ummantelung mit einem neuartigern Aufbau bereitgestellt wird, welcher insbesondere eine äusserst kompakte Bauweise aufweist. Dies bringt grosse Vorteile bei der Einführung des Katheters mit sich, da dieser aufgrund des geringen Aussendurchmessers bestmöglich durch Verengungen in einem Hohlorgan oder einem Gefäss geführt werden kann. Zudem lässt sich der erfindungsgemässe Katheter in ökonomischer Weise herstellen.

## Patentansprüche

1. Katheter (10, 11, 12) zur Einführung in ein menschliches oder tierisches Hohlorgan, umfassend eine nach vorne ragende Katheterspitze (40, 41, 42), wobei hinter der Katheterspitze (40, 41, 42) ein von einem expandierbaren röhrenförmigem Hohlkörper (60, 70) ummantelter Bereich angeordnet ist, wobei im ummantelten Bereich eine Drahtwendel (20, 21, 22) angeordnet ist, wobei die Drahtwendel (20) aus röntgendichtem Material besteht, **und wobei** mehrere benachbarte Windungen der Drahtwendel (20) aneinander anliegend angeordnet sind und so einen röntgendichten Bereich des Katheters bilden und zur Erzeugung eines röntgendurchlässigen Bereichs (20.2, 20.4) der Drahtwendel (20) weitere benachbarte Windungen berührungslos und voneinander beabstandet angeordnet sind, und die Drahtwendel (20) innerhalb eines im ummantelten Bereich angeordneten Hohlschafts (30) des Katheters (10) angeordnet ist, und
**dadurch gekennzeichnet, dass**
der Hohlschaft (30) derart an die Drahtwendel angeformt ist, dass eine äussere Oberfläche (30.1) des Hohlschafts (30) im Wesentlichen einer äusseren Kontur der Drahtwendel (20) entspricht.

2. Katheter (10) nach Anspruch 1, **dadurch gekennzeichnet, dass** eine Längsachse der Drahtwendel (20) koaxial zu einer Längsachse des Katheters (10) angeordnet ist und dass sich insbesondere die Drahtwendel (20) in einer Längsrichtung, welche parallel zur Längsachse des Katheters (10) verläuft, von einem vorderen Ende (60.1, 70.1) des expandierbaren röhrenförmigen Hohlkörpers (60, 70) bis an ein hinteres Ende (60.2, 70.2) des expandierbaren röhrenförmigen Hohlkörpers (60, 70) erstreckt.

3. Katheter (10) nach einem der Ansprüche 1 - 2, **dadurch gekennzeichnet, dass** es sich beim im ummantelten Bereich angeordneten Hohlschaft (30) des Katheters (10) um einen innersten Hohlschaft des Katheters (10) handelt.

4. Katheter (10) nach einem der Ansprüche 1 - 3, **dadurch gekennzeichnet, dass** die Drahtwendel (20) einen Aussendurchmesser von 0.2 - 0.5 mm und einen Drahtdurchmesser von 30 - 100 µm aufweist.

5. Katheter (10) nach einem der Ansprüche 1-4, **dadurch gekennzeichnet, dass** innerhalb der Drahtwendel (21) ein geradliniger Innendraht (41.2) angebracht ist, welcher in einem oder mehreren Verbindungsbereichen (91.1, 91.2, 91.3) punktuell mit der Drahtwendel (21) verbunden ist.

6. Katheter (11) nach Anspruch 5, **dadurch gekennzeichnet, dass** die Drahtwendel (22) und der geradlinige Innendraht (41.2) in den Verbindungsbereichen (91.1, 91.2, 91.3) mit einem röntgendichten Lot, insbesondere einem Silberlot, verlötet sind, wobei die Verbindungsbereiche (91.1, 91.2, 91.3) röntgendichte Bereiche des Katheters (11) bilden.

7. Katheter (10) nach einem der Ansprüche 1 - 6, **dadurch gekennzeichnet, dass** die Drahtwendel (20) aus röntgendichtem Material aus Platin, Gold oder Wolfram besteht.

8. Katheter (10) nach Anspruch 1, **dadurch gekennzeichnet, dass,** zwischen den einzelnen Windungen ein Abstand (L) vorliegt, welcher wenigstens dem zweifachen, insbesondere dem dreifachen, Drahtdurchmesser entspricht.

9. Katheter (10) nach Anspruch 1, **dadurch gekennzeichnet, dass** zur Längenmarkierung mehrere röntgendichte Bereiche (20.1, 20.3, 20.5) und mehrere röntgendurchlässige Bereiche (20.2, 20.4) der Drahtwendel in einer alternierenden Abfolge und insbesondere in regelmässigen Abständen angeordnet sind.

10. Katheter (10) nach einem der Ansprüche 1-9, **dadurch gekennzeichnet, dass** wenigstens zwei expandierbare röhrenförmige Hohlkörper (60, 70) vorliegen, welche koaxial übereinander liegend angeordnet sind.

11. Katheter (10) nach einem der Ansprüche 1 - 10, **dadurch gekennzeichnet, dass** als expandierbarer röhrenförmiger Hohlkörper (60) ein beaufschlagbarer Ballon angeordnet ist.

12. Katheter (10) nach Anspruch 11, **dadurch gekennzeichnet, dass** der Ballon (60) eine Beschichtung aufweist, wobei die Beschichtung wenigstens ein Arzneimittel enthält.

13. Katheter (10) nach einem der Ansprüche 1 - 12, **dadurch gekennzeichnet, dass** als expandierbarer röhrenförmiger Hohlkörper (70) ein medizinisches Implantat, insbesondere ein Stent, angeordnet ist, welcher bevorzugt an eine äussere Kontur der Drahtwendel (20) angeformt ist.

14. Katheter nach Anspruch 13, **dadurch gekennzeichnet, dass** das medizinische Implantat (70) eine Beschichtung (70.3) aufweist, wobei die Beschichtung (70.3) wenigstens ein Arzneimittel enthält.

15. Katheter (10) nach einem der Ansprüche 1 - 14, **dadurch gekennzeichnet, dass** die Katheterspitze (40) im Wesentlichen als flexible Schraubenfeder (40.1) ausgebildet ist.

16. Katheter (12), nach einem der Ansprüche 1 - 15, zur Einführung in ein menschliches oder tierisches Hohlorgan, weiter umfassend eine nach vorne ragende Katheterspitze (42) und einen Hohlschaft (32), wobei auf dem Hohlschaft (32) ein röntgendichter Ring (100), insbesondere aus Platin, Gold oder Wolfram, angeordnet ist, wobei der Hohlschaft (32) in einem Bereich vor und/oder hinter dem röntgendichten Ring (100) mehrere den Hohlschaft (32) umlaufende und von diesem abstehende rippenartige vorstände (32.2...32.6) aufweist.

17. Verfahren zur Herstellung eines Katheters (10, 11, 12), insbesondere eines Katheters nach einem der Ansprüche 1 - 16, wobei eine Drahtwendel (20, 21, 22) mit einem expandierbaren röhrenförmigen Hohlkörper (60, 70), insbesondere einem Stent und/oder einem beaufschlagbaren Ballon, umgeben wird, wobei die Drahtwendel (20) vor einem Anbringen des expandierbaren röhrenförmigen Hohlkörpes (60, 70) in einem ersten Bereich (20.1) gestaucht und in einem zweiten Bereich (20.2), welcher direkt an den ersten Bereich (20.1) angrenzt, gestreckt wird, und
**dadurch gekennzeichnet, dass**
die Drahtwendel (20) vor einem Anbringen des expandierbaren röhrenförmigen Hohlkörpers (60, 70) in einen Hohlschaft (30) des Katheters (10) eingeführt wird und anschliessend der Hohlschaft (30) an eine äussere Kontur der Drahtwendel (20) angeformt wird.

18. Verfahren nach Anspruch 17, **dadurch gekennzeichnet, dass** ein röntgendichter Ring (100) auf den Hohlschaft (32) aufgebracht wird und dass zur Bildung von rippenartigen Vorständen (32.2...32.6) anschliessend der Hohlschaft (32) in mehreren Bereichen vor dem röntgendichten Ring (100) und/oder in mehreren Bereichen hinter dem röntgendichten Ring (100) in einer Längsrichtung des Hohlschafts (32) gestaucht wird.

## Claims

1. Catheter (10, 11, 12) to be inserted into a hollow organ of a human or an animal, comprising a forwardly projecting catheter tip (40, 41, 42), wherein a region encased by an expandable tubular hollow body (60, 70) is arranged behind the catheter tip (40, 41, 42), wherein a wire helix (20, 21, 22) is arranged in the encased region, wherein the wire helix (20) consists of radiopaque material, and wherein a plurality of adjacent windings of the wire helix (20) are arranged abutting one another and thus form a radiopaque region of the catheter and further adjacent windings are arranged without touching and spaced apart for producing a radiolucent region (20.2, 20.4) of the wire helix (20), and the wire helix (20) is arranged within a hollow shaft (30) of the catheter (10) arranged within the encased region, and **characterised in that** the hollow shaft (30) is fitted to the wire helix such that an outer surface (30.1) of the hollow shaft (30) substantially corresponds to an outer contour of the wire helix (20).

2. Catheter (10) according to Claim 1, **characterized in that** a longitudinal axis of the wire helix (20) is arranged coaxially to a longitudinal axis of the catheter (10) and **in that**, more particularly, the wire helix (20) extends from a front end (60.1, 70.1) of the expandable tubular hollow body (60, 70) to a rear end (60.2, 70.2) of the expandable tubular hollow body (60, 70) in a longitudinal direction, which runs parallel to the longitudinal axis of the catheter (10).

3. Catheter (10) according to either of Claims 1 and 2, **characterized in that** the hollow shaft (30) of the catheter (10) arranged within the encased region is an innermost hollow shaft of the catheter (10).

4. Catheter (10) according to one of Claims 1-3, **characterized in that** the wire helix (20) has an external diameter of 0.2-0.5 mm and a wire diameter of 30-100 µm.

5. Catheter (10) according to one of Claims 1-4, **characterized in that** a straight-line inner wire (41.2) is attached within the wire helix (21), with the former being connected to the wire helix (21) in a punctiform fashion at one or more connection regions (91.1, 91.2, 91.3) .

6. Catheter (11) according to Claim 5, **characterized in that** the wire helix (22) and the straight-line inner wire (41.2) are brazed together in the connection regions (91.1, 91.2, 91.3) using a radiopaque solder, more particularly a silver solder, wherein the connection regions (91.1, 91.2, 91.3) form radiopaque regions of the catheter (11).

7. Catheter (10) according to one of Claims 1-6, **characterized in that** the wire helix (20) consists of radiopaque material made of platinum, gold or tungsten.

8. Catheter (10) according to Claim 1, **characterized in that** a spacing (L) is between the individual windings, corresponding to at least double, more particularly triple, the wire diameter.

9. Catheter (10) according to Claim 1, **characterized in that** a plurality of radiopaque regions (20.1, 20.3, 20.5) and a plurality of radiolucent regions (20.2, 20.4) of the wire helix are arranged alternately and more particularly at regular intervals for marking the length.

10. Catheter (10) according to one of Claims 1-9, **characterized in that** there are at least two expandable tubular hollow bodies (60, 70) arranged lying coaxially above one another.

11. Catheter (10) according to one of Claims 1-10, **characterized in that** an actuatable balloon is arranged as expandable tubular hollow body (60).

12. Catheter (10) according to Claim 11, **characterized in that** the balloon (60) has a coating, wherein the coating contains at least one medicament.

13. Catheter (10) according to one of Claims 1-12, **characterized in that** a medical implant, more particularly a stent, is arranged as an expandable tubular hollow body (70), which is preferably fitted to an outer contour of the wire helix (20).

14. Catheter according to Claim 13, **characterized in that** the medical implant (70) has a coating (70.3), wherein the coating (70.3) contains at least one medicament.

15. Catheter (10) according to one of Claims 1-14, **characterized in that** the catheter tip (40) is basically designed as a flexible helical spring (40.1).

16. Catheter (12), according to one of Claims 1-15, to be inserted into a hollow organ of a human or an animal, further comprising a forwardly projecting catheter tip (42) and a hollow shaft (32), wherein a radiopaque ring (100), more particularly made of platinum, gold or tungsten, is arranged on the hollow shaft (32), wherein the hollow shaft (32) has a plurality of rib-like projections (32.2...32.6) surrounding the hollow shaft (32) and projecting from the latter in a region in front of and/or behind the radiopaque ring (100).

17. Method for producing a catheter (10, 11, 12), more particularly a catheter according to one of Claims 1-16, wherein a wire helix (20, 21, 22) is surrounded by an expandable, tubular hollow body (60, 70), more particularly a stent and/or an actuatable balloon, wherein the wire helix (20) is compressed in a first region (20.1) and stretched in a second region (20.2), which directly adjoins the first region (20.1), prior to an application of the expandable tubular hollow body (60, 70), and **characterized in that**
the wire helix (20) is inserted into a hollow shaft (30) of the catheter (10) prior to an application of the expandable tubular hollow body (60, 70) and the hollow shaft (30) is subsequently fitted to an outer contour of the wire helix (20).

18. Method according to Claim 17, **characterized in that** a radiopaque ring (100) is attached to the hollow shaft (32) and **in that** the hollow shaft (32) is subsequently compressed in a longitudinal direction of the hollow shaft (32) in a plurality of regions in front of the radiopaque ring (100) and/or in a plurality of regions behind the radiopaque ring (100) for the formation of rib-like projections (32.2...32.6).

## Revendications

1. Cathéter (10, 11, 12) pour l'introduction dans un organe creux humain ou animal, comprenant une pointe de cathéter saillant vers l'avant (40, 41, 42), une région enveloppée par un corps creux expansible en forme de tube (60, 70) étant disposée derrière la pointe de cathéter (40, 41, 42), une spirale en fil métallique (20, 21, 22) étant disposée dans la région enveloppée, la spirale en fil métallique (20) se composant d'un matériau radio-opaque et plusieurs spires adjacentes de la spirale en fil métallique (20) étant disposées de manière à s'appliquer les unes contre les autres et formant ainsi une région radio-opaque du cathéter et, pour produire une région radio-transparente (20.2, 20.4) de la spirale en fil métallique (20), d'autres spires adjacentes étant disposées sans contact et à distance les unes des autres, et la spirale en fil métallique (20) étant disposée à l'intérieur d'une tige creuse (30) du cathéter (10) disposée dans la région enveloppée et
**caractérisé en ce que**
la tige creuse (30) est façonnée au niveau de la spirale en fil métallique de telle sorte qu'une surface extérieure (30.1) de la tige creuse (30) corresponde essentiellement à un contour extérieur de la spirale en fil métallique (20).

2. Cathéter (10) selon la revendication 1, **caractérisé en ce qu'**un axe longitudinal de la spirale en fil métallique (20) est disposé coaxialement à un axe longitudinal du cathéter (10) et **en ce que** la spirale en fil métallique (20) s'étend notamment dans une direction longitudinale qui s'étend parallèlement à l'axe longitudinal du cathéter (10), depuis une extrémité avant (60.1, 70.1) du corps creux expansible en forme de tube (60, 70) jusqu'à une extrémité arrière (60.2, 70.2) du corps creux expansible en forme de tube (60, 70).

3. Cathéter (10) selon l'une quelconque des revendications 1 et 2, **caractérisé en ce que** la tige creuse (30) du cathéter (10) disposée dans la région enveloppée est une tige creuse la plus intérieure du cathéter (10).

4. Cathéter (10) selon l'une quelconque des revendications 1 à 3, **caractérisé en ce que** la spirale en fil métallique (20) présente un diamètre extérieur de 0,2 à 0,5 mm et un diamètre de fil métallique de 30 à 100 µm.

5. Cathéter (10) selon l'une quelconque des revendications 1 à 4, **caractérisé en ce qu'à** l'intérieur de la spirale en fil métallique (21) est installé un fil métallique intérieur rectiligne (41.2) qui est connecté dans une ou plusieurs régions de connexion (91.1, 91.2, 91.3) ponctuellement à la spirale en fil métallique (21) .

6. Cathéter (11) selon la revendication 5, **caractérisé en ce que** la spirale en fil métallique (22) et le fil intérieur rectiligne (41.2) sont brasés dans les régions de connexion (91.1, 91.2, 91.3) avec une brasure radio-opaque, en particulier une brasure d'argent, les régions de connexion (91.1, 91.2, 91.3) formant des régions radio-opaques du cathéter (11).

7. Cathéter (10) selon l'une quelconque des revendications 1 à 6, **caractérisé en ce que** la spirale en fil métallique (20) se compose de matériau radio-opaque constitué de platine, d'or ou de tungstène.

8. Cathéter (10) selon la revendication 1, **caractérisé en ce qu'**entre les spires individuelles existe un espace (L) qui correspond au moins au double, en particulier au triple, du diamètre du fil métallique.

9. Cathéter (10) selon la revendication 1, **caractérisé en ce que** pour le marquage en longueur, plusieurs régions radio-opaques (20.1, 20.3, 20.5) et plusieurs régions radio-transparentes (20.2, 20.4) de la spirale en fil métallique sont disposées en succession alternée et notamment à intervalles réguliers.

10. Cathéter (10) selon l'une quelconque des revendications 1 à 9, **caractérisé en ce qu'**au moins deux corps creux expansibles en forme de tube (60, 70) sont prévus, lesquels sont disposés de manière à être situés coaxialement l'un au-dessus de l'autre.

11. Cathéter (10) selon l'une quelconque des revendications 1 à 10, **caractérisé en ce qu'**un ballonnet pouvant être sollicité est prévu en tant que corps creux expansible en forme de tube (60).

12. Cathéter (10) selon la revendication 11, **caractérisé en ce que** le ballonnet (60) présente un revêtement, le revêtement contenant au moins un médicament.

13. Cathéter (10) selon l'une quelconque des revendications 1 à 12, **caractérisé en ce qu'**un implant médical, en particulier un stent, est prévu(e) en tant que corps creux expansible en forme de tube (70), qui est façonné de préférence au niveau d'un contour extérieur de la spirale en fil métallique (20).

14. Cathéter selon la revendication 13, **caractérisé en ce que** l'implant médical (70) présente un revêtement (70.3), le revêtement (70.3) contenant au moins un médicament.

15. Cathéter (10) selon l'une quelconque des revendications 1 à 14, **caractérisé en ce que** la pointe de cathéter (40) est réalisée essentiellement sous forme de ressort hélicoïdal flexible (40.1).

16. Cathéter (12) selon l'une quelconque des revendications 1 à 15, pour l'introduction dans un organe creux humain ou animal, comprenant en outre une pointe de cathéter saillant vers l'avant (42) et une tige creuse (32), une bague radio-opaque (100), en particulier en platine, en or ou en tungstène, étant disposée sur la tige creuse (32), la tige creuse (32) présentant, dans une région devant et/ou derrière la bague radio-opaque (100), plusieurs saillies de type nervure (32.2...32.6) entourant la tige creuse (32) et faisant saillie depuis celle-ci.

17. Procédé de fabrication d'un cathéter (10, 11, 12), en particulier d'un cathéter selon l'une quelconque des revendications 1 à 16, dans lequel une spirale en fil métallique (20, 21, 22) est entourée par un corps creux expansible en forme de tube (60, 70), en particulier un stent et/ou un ballonnet pouvant être sollicité, la spirale en fil métallique (20), avant l'installation du corps creux expansible en forme de tube (60, 70), étant comprimée dans une première région (20.1) et étant étirée dans une deuxième région (20.2) qui est directement adjacente à la première région (20.1),
et
**caractérisé en ce que**
la spirale en fil métallique (20), avant une installation du corps creux expansible en forme de tube (60, 70), est introduite dans une tige creuse (30) du cathéter (10) et ensuite la tige creuse (30) est façonnée au niveau d'un contour extérieur de la spirale en fil métallique (20).

18. Procédé selon la revendication 17, **caractérisé en ce qu'**une bague radio-opaque (100) est placée sur la tige creuse (32) et **en ce que** pour former des saillies de type nervure (32.2...32.6), la tige creuse (32) est ensuite comprimée dans plusieurs régions devant la bague radio-opaque (100) et/ou dans plusieurs régions derrière la bague radio-opaque (100) dans une direction longitudinale de la tige creuse (32).
